# EUROPEAN PATENT SPECIFICATION

(11) **EP 4 061 276 B1**
(45) Date of publication and mention of the grant of the patent: **04.09.2024**
(21) Application number: 20824371.7
(22) Date of filing: 13.11.2020
(51) Int. Cl.: A61C 13/00, A61C 13/01, A61C 13/10, B33Y 10/00, B33Y 70/00, B33Y 80/00, C08F 290/06, C08G 18/00, C08G 18/68, C08L 51/08

(54) **PARTIAL DENTURES AND METHODS OF MAKING THE SAME**
TEILPROTHESEN UND VERFAHREN ZUM HERSTELLEN DERSELBEN
PROTHÈSES DENTAIRES PARTIELLES ET LEURS PROCÉDÉS DE FABRICATION

(30) Priority: 18.11.2019 US 201962936895 P
(43) Date of publication of application: 28.09.2022
(73) Proprietor: CARBON, INC., Redwood City, California 94063 (US)
(72) Inventor: HERRING, Marie, K., Palo Alto, CA 94306 (US)
(74) Representative: Marks & Clerk LLP
(86) International application number: PCT/US2020/060391
(87) International publication number: WO 2021/101801

(56) References cited:
- EP-A1- 3 708 336
- WO-A1-2018/038056
- US-A1- 2017 135 790

## Description

### Field of the Invention

Partial dentures, partial denture bases, and methods of making the same are described.

### Background of the Invention

People with missing teeth use dentures to aid in chewing, improve their appearance, and enhance their speaking ability. Full dentures are used when all teeth are missing, and partial dentures are used when some teeth remain. While it is generally necessary for full dentures to be rigid, partial dentures can be either rigid or flexible, and flexible partial dentures have become more popular because they may flex around the remaining teeth and do not always require invasive procedures. In addition, flexible partial dentures can in some cases be provided to a patient on a same-day basis.

With the demand for flexible partial dentures increasing, there is a need for new approaches to for the prompt manufacture of such dentures in a cost-effective manner.

For example document WO2018038056 discloses a method of additive manufacturing mouth guards, occlusal splints or denture bases. In some cases a post-curing step by light irradiation or by heat is performed.

### Summary of the Invention

Provided herein is a method of making a flexible partial denture, which may include: *(a)* producing a partial denture base by additive manufacturing (*e.g.*, bottom-up or top-down stereolithography) from a dual cure resin, with the dual cure resin comprising a light polymerizable component and a heat polymerizable component, with the denture base including at least one tooth socket comprising the dual cure resin in uncured or partially cured form; *(b)* inserting an acrylic artificial tooth into each at least one tooth socket to produce an assembled intermediate product; and then (c) baking said assembled intermediate product for a time sufficient to cure said heat polymerizable component in said denture base and the dual cure resin in uncured or partially cured form in said at least one tooth socket, bond said tooth into each at least one socket with said cure, and produce said flexible partial denture.

In some embodiments, the resin comprises or consists essentially of a mixture of (i) a reactive blocked prepolymer comprising a compound of the formula A-X-A, where X is a hydrocarbyl group and each A is an independently selected substituent of Formula X: 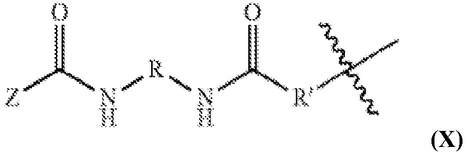 where R is a hydrocarbyl group, R' is O or NH, and Z is a blocking group, said blocking group having a reactive epoxy, alkene, alkyne, or thiol terminal group, (ii) a chain extender (e.g., a polyamine and/or polyol chain extender), (iii) a photoinitiator, (iv) a reactive diluent, (v) a reactive blocked diisocyanate, (vi) optionally, but in some embodiments preferably, a reactive crosslinker; (vii) optionally, but in some embodiments preferably, at least one pigment or dye; and (viii) optionally, but in some embodiments preferably, an ultraviolet light absorber.

Also provided is a flexible partial denture produced by the process described herein.

Further provided is a resin composition comprising or consisting essentially of a mixture of (i) a reactive blocked prepolymer comprising a compound of the formula A-X-A, where X is a hydrocarbyl group and each A is an independently selected substituent of Formula X: 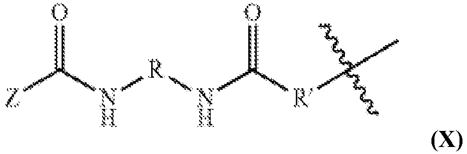 where R is a hydrocarbyl group, R' is O or NH, and Z is a blocking group, said blocking group having a reactive epoxy, alkene, alkyne, or thiol terminal group, (ii) a chain extender (*e.g.* MACM), (iii) a photoinitiator, (iv) a reactive diluent, (v) a reactive blocked diisocyanate, (vi) optionally, but in some embodiments preferably, a reactive crosslinker; (vii) optionally, but in some embodiments preferably, at least one pigment or dye; and (viii) optionally, but in some embodiments preferably, an ultraviolet light absorber.

### Brief Description of the Drawings

**Figure 1** is an exploded view showing a flexible polymeric denture base (13) and corresponding acrylic artificial teeth (11) to be inserted into the denture base sockets (12).
**Figure 2** shows the denture base (13) of Figure 1, with the acrylic artificial teeth (11) partially inserted therein. The teeth will be fully inserted before the assembled intermediate object is baked.
**Figure 3** shows an assembled flexible polymer partial denture including both base (13) and fully inserted teeth (11).
**Figure 4** shows the modulus of elasticity of objects made from resins described herein.
**Figure 5** shows the impact strength of objects made from resins described herein.

### Detailed Description of Illustrative Embodiments

The present invention is now described more fully hereinafter with reference to the accompanying drawings, in which embodiments of the invention are shown. This invention may, however, be embodied in many different forms and should not be construed as limited to the embodiments set forth herein; rather the scope of the invention is defined by the claims.

As used herein, the term "and/or" includes any and all possible combinations of one or more of the associated listed items, as well as the lack of combinations when interpreted in the alternative ("or").

### 1. RESINS FOR ADDITIVE MANUFACTURING OF DENTURE BASES.

While the denture bases described herein can be made with any suitable resin, dual cure resins are currently preferred for carrying out the present invention. Such resins are known and described in, for example, US Patent Nos. 9,676,963, 9,453,142 and 9,598,606 to Rolland et al. and US Patent No. 10,316,213 to Amdt et al.

In general, such resins comprise a light polymerizable component and a heat polymerizable component. The heat polymerizable component preferably comprises precursors to a polyurethane, polyurea, or copolymer thereof. Examples of suitable dual cure resins include, but are not limited to, Carbon Inc. flexible polyurethane dual cure resins, available from Carbon, Inc., 1089 Mills Way, Redwood City, California 94063 USA.

***Light-polymerizable monomers and*/*****or prepolymers.*** Sometimes also referred to as "Part A" of the resin, these are monomers and/or prepolymers that can be polymerized by exposure to actinic radiation or light. This resin can have a functionality of 2 or higher (though a resin with a functionality of 1 can also be used when the polymer does not dissolve in its monomer). A purpose of Part A is to "lock" the shape of the object being formed or create a scaffold for the one or more additional components (e.g., Part B). Importantly, Part A is present at or above the minimum quantity needed to maintain the shape of the object being formed after the initial solidification during photolithography.

Examples of suitable reactive end groups suitable for Part A constituents, monomers, or prepolymers include, but are not limited to: acrylates, methacrylates, α-olefins, N-vinyls, acrylamides, methacrylamides, styrenics, epoxides, thiols, 1,3-dienes, vinyl halides, acrylonitriles, vinyl esters, maleimides, and vinyl ethers.

An aspect of the solidification of Part A is that it provides a scaffold in which a second reactive resin component, termed "Part B," can solidify during a second step, as discussed further below.

***Heat-polymerizable monomers and*/*or prepolymers.*** Sometimes also referred to as "Part B", these constituents may comprise, consist of or consist essentially of a mix of monomers and/or prepolymers that possess reactive end groups that participate in a second solidification reaction during or after the Part A solidification reaction, preferably a heat cure (e.g., by baking). In some embodiments, the second component of the dual cure resin comprises precursors to a polyurethane, polyurea, or copolymer thereof (e.g., poly(urethane-urea)).

Examples of dual cure photopolymerizable reactive prepolymers include isocyanate or blocked isocyanate-containing prepolymers. A blocked isocyanate is a group that can be deblocked or is otherwise available for reaction as an isocyanate upon heating, such as by a urethane reaction of an isocyanate with alcohol. *See, e.g.*, U.S. Patent No. 3,442,974 to Bremmer; U.S. Patent No. 3,454,621 to Engel; Lee et al., "Thermal Decomposition Behaviour of Blocked Diisocyanates Derived from Mixture of Blocking Agents," Macromolecular Research, 13(5):427-434 (2005).

"Isocyanate" as used herein includes diisocyanate, polyisocyanate, and branched isocyanate. "Diisocyanate" and "polyisocyanate" are used interchangeably herein and refer to aliphatic, cycloaliphatic, and aromatic isocyanates that have at least 2, or in some embodiments more than 2, isocyanate (NCO) groups per molecule, on average. In some embodiments, the isocyanates have, on average, 2.1, 2.3, 2.5, 2.8, or 3 isocyanate groups per molecule, up to 6, 8 or 10 or more isocyanate groups per molecule, on average. In some embodiments, the isocyanates may be a hyperbranched or dendrimeric isocyanate (e.g., containing more than 10 isocyanate groups per molecule, on average, up to 100 or 200 more more isocyanate groups per molecule, on average). Common examples of suitable isocyanates include, but are not limited to, methylene diphenyl diisocyanate (MDI), toluene diisocyanate (TDI)), para-phenyl diisocyanate (PPDI), 4,4'-dicyclohexylmethane- diisocyanate (HMDI), hexamethylene diisocyanate (HDI), isophorone diisocyanate (IPDI), triphenylmethane-4,4'4"-triisocyanate, tolune-2,4,6- triyl triisocyanate, 1,3,5-triazine-2,4,6-triisocyanate, ethyl ester L-lysine triisocyanate, etc., including combinations thereof. Numerous additional examples are known and are described in, for example, US Patent Nos. 9,200,108; 8,378,053; 7,144,955; 4,075,151, 3,932,342, and in US Patent Application Publication Nos. US 20040067318 and US 2014037: 406.

The blocking group may optionally have a reactive terminal group (e.g., a polymerizable end group such as an epoxy, alkene, alkyne, or thiol end group, for example an ethylenically unsaturated end group such as a vinyl ether).

In some embodiments, the dual cure resin includes a UV-curable (meth)acrylate blocked polyurethane/polyurea (ABPU). Such resins are described in, for example, Rolland et al., US Patent Nos. 9,676,963, 9,598,606, and 9,453,142.

In some embodiments, the isocyanate may be blocked with an amine methacrylate blocking agent (e.g., tertiary-butylaminoethyl methacrylate (TBAEMA), tertiary pentylaminoethyl methacrylate (TPAEMA), tertiary hexylaminoethyl methacrylate (THAEMA), tertiary-butylaminopropyl methacrylate (TBAPMA), maleimide, and mixtures thereof (*see, e.g.*, US Patent Application Publication No. 20130202392)). Note that these could be used as diluents, as well.

***Chain extenders.*** Chain extenders are generally linear compounds having di- or polyfunctional ends that can react with a monomer/prepolymer or crosslinked photopolymerized polymer intermediate as taught herein. Examples include, but are not limited to, diol or amine chain extenders, which can react with isocyanates of a de-blocked diisocynate-containing polymer.

Examples of diol or polyol chain extenders include, but are not limited to, ethylene glycol, diethylene glycol, triethylene glycol, tetraethylene glycol, propylene glycol, dipropylene glycol, tripropylene glycol, 1,3-propanediol, 1,3-butanediol, 1,4-butanediol, neopentyl glycol, 1,6-hexanediol, 1,4-cyclohexanedimethanol, hydroquinone bis(2-hydroxyethyl) ether (HQEE), glycerol, trimethylolpropane, 1,2,6-hexanetriol, and pentaerythritol. Natural oil polyols (biopolyols) may also be used. Such polyols may be derived, *e.g.*, from vegetable oils (triglycerides), such as soybean oil, by known techniques. *See, e.g.*, U.S. Patent No. 6,433,121 to Petrovic et al.

Examples of diamine or polyamine chain extenders include, but are not limited to, aliphatic, aromatic, and mixed aliphatic and aromatic, polyamines, such as diamines (for example, 4,4'-methylenedicyclohexanamine (PACM), 4,4'-methylenebis(2-methylcyclohexylamine) (MACM), ethylene diamine, isophorone diamine, diethyltoluenediamine), and polyetheramines (for example JEFFAMINE^{®} from Huntsman Corporation).

***Diluents.*** Diluents as known in the art are compounds used to reduce viscosity in a resin composition. Reactive diluents undergo reaction to become part of the polymeric network. In some embodiments, the reactive diluent may react at approximately the same rate as other reactive monomers and/or prepolymers in the composition. Reactive diluents may include aliphatic reactive diluents, aromatic reactive diluents, and cycloaliphatic reactive diluents. Examples include, but are not limited to, isobomyl acrylate, isobomyl methacrylate, lauryl acrylate, lauryl methacrylate, 2-ethyl hexyl methacarylate, 2-ethyl hexyl acrylate, di(ethylene glycol) methyl ether methacrylate, phenoxyethyl methacrylate, hydroxyethyl acrylate, hydroxyethyl methacrylate, dimethyl(aminoethyl) methacrylate, butyl acrylate, butyl methacrylate, cyclohexyl methacrylate, tetrahydrofurfuryl methacrylate, ethylene glycol dimethacrylate, hexanediol dimethacrylate, and tert-butylaminoethyl methacrylate.

In some embodiments, crosslinking diluents (i.e., having a functionality of 2 or more) may be used. Examples of such crosslinking diluents include but are not limited to a dimethacrylate such as neopentyl glycol dimethacrylate (NPGDMA), ethylene glycol dimethacrylate (EGDMA), etc.

In some embodiments, crosslinking agents may be included in the resin, which agent may be a crosslinking diluent (as noted above), or an agent that increases the resin viscosity. Examples of such crosslinking agents include but are not limited to a urethane dimethacrylate such as isophorone urethane dimethacrylate (UDMA), available from Esstech Inc., Essington, Pennsylvania, USA as their product X-851-1066.

Photoinitiators useful in the present invention include, but are not limited to, diphenyl(2,4,6-trimethylbenzoyl)phosphine oxide (TPO), Phenylbis(2,4,6-trimethylbenzoyl)phosphine oxide (PPO), 2-isopropylthioxanthone and/or 4-isopropylthioxanthone (ITX), etc.

The liquid resin can have additional ingredients solubilized therein, including pigments, dyes, active compounds or pharmaceutical compounds, detectable compounds (*e.g.*, fluorescent, phosphorescent, radioactive), etc.

Ultraviolet light absorbers that can be used in the present invention include polysubstituted linear polyacenes such as described in G. Robbins and S. Pollack, PCT Patent Application Publication No. WO/2019/060239 (published 28 March 2019).

### 2. ADDITIVE MANUFACTURING OF DENTURE BASES.

Denture bases can be produced in a "green" form from resins described above by additive manufacturing techniques.

Techniques for producing an intermediate object, or "green" intermediate, from such resins by additive manufacturing are known. Suitable techniques include bottom-up and top-down additive manufacturing, generally known as stereolithography. Such methods are known and described in, for example, U.S. Patent No. 5,236,637 to Hull, US Patent Nos. 5,391,072 and 5,529,473 to Lawton, U.S. Patent No. 7,438,846 to John, US Patent No. 7,892,474 to Shkolnik, U.S. Patent No. 8,110,135 to El-Siblani, U.S. Patent Application Publication No. 2013/0292862 to Joyce, and US Patent Application Publication No. 2013/0295212 to Chen et al.

In some embodiments, the additive manufacturing step is carried out by one of the family of methods sometimes referred to as continuous liquid interface production (CLIP). CLIP is known and described in, for example, US Patent Nos. 9,211,678; 9,205,601; 9,216,546; and others; in J. Tumbleston et al., Continuous liquid interface production of 3D Objects, Science 347, 1349-1352 (2015); and in R Janusziewcz et al., Layerless fabrication with continuous liquid interface production, Proc. Natl. Acad Sci. USA 113, 11703-11708 (October 18, 2016). Other examples of methods and apparatus for carrying out particular embodiments of suitable methods of making include, but are not limited to: Batchelder et al., US Patent Application Pub. No. US 2017/0129169; Sun and Lichkus, US Patent Application Pub. No. US 2016/0288376; Willis et al., US Patent Application Pub. No. US 2015/0360419; Lin et al., US Patent Application Pub. No. US 2015/0331402; D. Castanon, US Patent Application Pub. No. US 2017/0129167; L. Robeson et al., PCT Patent Pub. No. WO 2015/164234 (see also US Patent Nos. 10,259,171 and 10,434,706); C. Mirkin et al., PCT Patent Pub. No. WO 2017/210298 (see also US Pat. App. US 2019/0160733); B. Feller, US Pat App. Pub. No. US 2018/0243976 (published Aug 30, 2018); M. Panzer and J. Tumbleston, US Pat App Pub. No. US 2018/0126630 (published May 10, 2018); and K. Willis and B. Adzima, US Pat App Pub. No. US 2018/0290374 (Oct. 11, 2018).

Once the denture base has been produced in a "green" form as described above, it may be optionally cleaned (e.g., by wiping, blowing, spinning, washing, etc.), and used to assemble a denture as discussed further below. If cleaned, in some embodiments it may be cleaned under conditions in which uncured or partially-cured resin is retained in the denture base sockets (or added back to denture base sockets) to bond the artificial teeth to the finished object as discussed below.

### 3. PRODUCTION OF PARTIAL DENTURES.

The same dual cure resin as used to form the denture base can advantageously be used to bond acrylic artificial teeth into that base. Partial dentures as used herein can include a plurality of teeth, for example 2, 3, or 4 or more teeth, but not an entire set of teeth.

As noted above, in some embodiments the denture base in green form may be cleaned under conditions in which uncured or partially-cured resin is retained in the denture base sockets (or added back to denture base sockets) to bond the artificial teeth to the finished object as discussed below. Additional uncured resin may be added to the sockets, if desired. Alternatively, the denture base may be cleaned to remove most or all of the residual resin, followed by addition of uncured resin to the sockets before or during insertion of the artificial teeth.

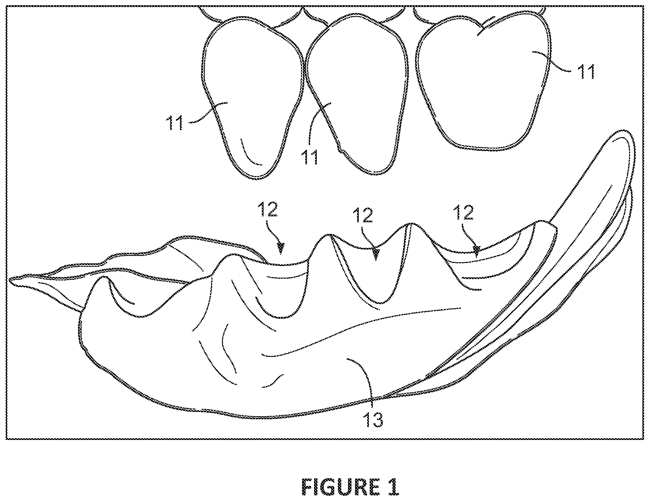

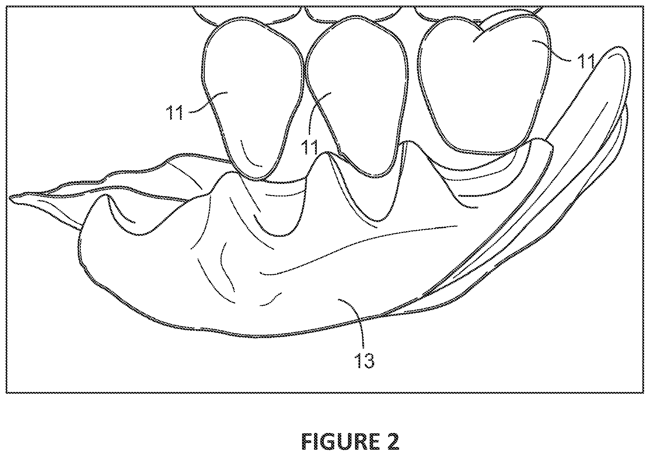

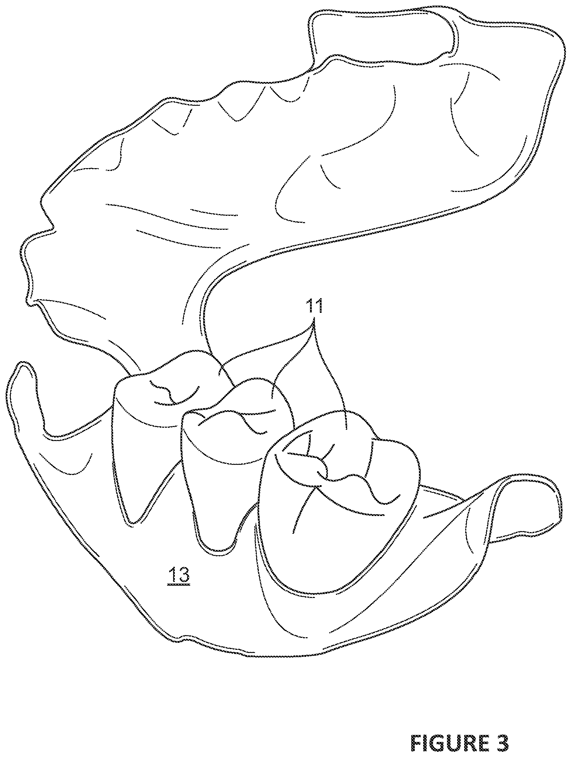

As shown in **Figures 1-3****,** acrylic artificial teeth **11** are inserted into the sockets **12** of a denture base **13**, while the base is in a "green" form, to produce an assembled intermediate. That intermediate can then optionally be cleaned (in like manner as described above), and then heated or baked in accordance with known techniques to both further cure the base (and achieve the desired tensile properties therein) and bond the artificial teeth to the base.

In some embodiments, the denture base with inserted artificial teeth and uncured resin in the sockets is subjected to a "flood" UV cure to polymerize the resin, optionally prior to the baking step.

Additional processing steps such as removal of supports, grinding or polishing of edges or surfaces and the like, can be performed before or after assembly of the complete denture, in accordance with known techniques.

Any type of acrylic artificial tooth can be used in the methods and products described herein, including those formed from a conventional acrylic resin and those formed from a modified acrylic resin. The resin and artificial tooth can incorporate cross-linking agents to enhance mechanical strength, can include copolymers or poly(methyl methacrylate), can include an interpenetrating polymer network, etc. In some embodiments the teeth can be manufactured using multilayered or double cross-linked material. The tooth can include additional constituents such as ultra-high molecular weight polyethylene, inorganic microparticle fillers (such as a microfiller-reinforced polyacrylic), and can include layers with a pearly effect to provide a more natural appearance. *See generally* K. Neppelenbroek et al., Surface properties of multilayered, acrylic resin artificial teeth after immersion in staining beverages, J. Appl. Oral Sci. 23(4): 376-82 (2015); *see also* US Patent Nos. 10,085,923; 9,861,557; and 5,070,165. Commercial sources for acrylic artificial teeth include: Ivoclar Vivadent Inc., 175 Pineview Drive, Amherst, NY 14228 USA; Candulor AG, Boulevard Lilienthal 8, 8152 Glattpark (Opfikon), Switzerland; Kulzer GmbH, 4315 S. Lafayette Blvd., South Bend, IN 46614 USA; and others. In some embodiments the artificial tooth itself may be produced by additive manufacturing, such as (for example) a DENTCA^{™} artificial tooth. Y-J Chung et al., 3D printing of resin material for denture artificial teeth: Chipping and indirect tensile fracture resistance, Materials (Basel) 11(10): 1798 (2018).

In some embodiments, the flexible partial denture base after baking has a tensile modulus of from 300 to 2000 MPa (e.g., with ASTM D638: Tensile Testing) (e.g., 500-1500 MPa, or 700-1200 MPa), and/or an Izod impact strength of from 10 to 500 J/m² (e.g., with ASTM D256: Izod Impact Testing) (e.g., 500-400 J/m², or 100-300 J/m²).

### EXAMPLES

Non-limiting examples are given below, where the following abbreviations are used:
"ABPU" means (meth)acrylate blocked polyurethane (typically, UV-curable (meth)acrylate blocked polyurethane prepolymer)
"TB" or "TBAEMA" means 2-(tert-butylamino)ethyl methacrylate.
"IBOMA" means isobomyl methacrylate.
"MACM" means di(4-amino-3-methylcyclohexyl)methane.
"IPDI" means isophorone diisocyanate.
"HMDI" means bis-(4-isocyanatocyclohexyl)methane.
"PTMO" means poly(tetramethylene oxide).
"PO3G" means poly(oxytrimethylene)glycol, a bio-polymer made from 1,3-propanediol. "2K" means 2000 molecular weight.
"NPGDMA" means neopentyl glycol dimethacrylate (a resin viscosity-lowering cross-linker).
"DBA" means 9,10-dibromoanthracene (an ultraviolet light absorber).
"TPO" means diphenyl(2,4,6-trimethylbenzoyl)phosphine oxide (a photoinitiator).
"UDMA" means urethane dimethacrylate (a resin viscosity-increasing cross-linker), such as isophorone urethane dimethacrylate (HEMA-IPDI-HEMA), available from Esstech Inc., Essington, Pennsylvania, USA as their product X-851-1066.
"HEMA" means hydroxymethyl methacrylate.
"D2000" means JEFFAMINE^{®} D-2000 polyoxypropylenediamine, available from Huntsman Corp.

### EXAMPLES 1-6

### Preparation of Resin Formulations

Resin formulations were mixed by first adding NPGDMA, IBOMA and UDMA to a THINKY^{™} mixer cup with the TPO and DBA, then mixing at 2000 RPM until all the solids were dissolved (~10 minutes). Then the ABPU and any pigment components were added and mixed for 2 minutes at 2000 RPM. Finally, the amine components (MACM and, where present, D2000) were added and mixed for 2 minutes.

### EXAMPLE 1

### Resin Formulation 1

This resin was prepared in the manner described above from the ingredients set forth in Table 1 below. The specific ABPU was prepared from the constituent ingredients in the molar ratios indicated in the table in accordance with known techniques such as given in US Patent No. 9,453,142 to Rolland et al.

| **TABLE 1** | |
|---|---|
| **Ingredient** | **Amount** |
| ABPU (IPDI-PTMO 2.9K-TB, 3.56:1:5.13) | 74.4% |
| NPGDMA | 9.0% |
| UDMA | 6.0% |
| TPO | 0.8% |
| DBA | 0.15% |
| MACM | 9.7% |

### EXAMPLE 2

### Resin Formulation 2

This resin was prepared in the manner described above from the ingredients set forth in **Table 2** below. The specific ABPU was prepared from the constituent ingredients in the molar ratios indicated in the table in accordance with known techniques such as given in US Patent No. 9,453,142 to Rolland et al.

| **TABLE 2** | |
|---|---|
| **Ingredient** | **Amount** |
| ABPU (IPDI-PTMO 2.9K-TB, 3.56:1:5.13) | 58% |
| NPGDMA | 10.4% |
| UDMA | 8.1% |
| IBOMA | 15% |
| TPO | 0.8% |
| DBA | 0.15% |
| MACM | 7.6% |

### EXAMPLE 3

### Resin Formulation 3

This resin was prepared in the manner described above from the ingredients set forth in **Table 3** below. The specific ABPU was prepared from the constituent ingredients in the molar ratios indicated in the table in accordance with known techniques such as given in US Patent No. 9,453,142 to Rolland et al.

| **TABLE 3** | |
|---|---|
| **Ingredient** | **Amount** |
| ABPU | 57.7% |
| (IPDI-PO3G 2K-TB, 2.83:1:3.66) | |
| NPGDMA | 9.4% |
| UDMA | 9.4% |
| IBOMA | 14.9% |
| TPO | 0.8% |
| DBA | 0.1% |
| MACM | 7.6% |
| FD&C Red 40 Lake | 0.0038% |
| FD&C Blue 2 Lake | 0.0162% |

### EXAMPLE 4

### Resin Formulation 4

This resin was prepared in the manner described above from the ingredients set forth in **Table 4** below. The specific ABPU was prepared from the constituent ingredients in the molar ratios indicated in the table in accordance with known techniques such as given in US Patent No. 9,453,142 to Rolland et al.

| **TABLE 4** | |
|---|---|
| **Ingredient** | **Amount** |
| ABPU (IPDI-PO3G 2K-TB, 2.83:1:3.66) | 52.6% |
| NPGDMA | 10% |
| UDMA | 14.7% |
| IBOMA | 15% |
| TPO | 0.8% |
| DBA | 0.1% |
| MACM | 6.9% |
| FD&C Red 40 Lake | 0.0038% |
| FD&C Blue 2 Lake | 0.0162% |

### EXAMPLE5

### Resin Formulation 5

This resin was prepared in the manner described above from the ingredients set forth in **Table 5** below. The specific ABPU was prepared from the constituent ingredients in the molar ratios indicated in the table in accordance with known techniques such as given in US Patent No. 9,453,142 to Rolland et al.

| **TABLE 5** | |
|---|---|
| **Ingredient** | **Amount** |
| ABPU (IPDI-PTMO 2K-TB, 2.7-1-3.4) | 57.8% |
| NPGDMA | 9.4% |
| UDMA | 9.4% |
| IBOMA | 14.9% |
| TPO | 0.8% |
| DBA | 0.1% |
| MACM | 7.3% |
| FD&C Red 40 Lake | 0.01% |

### EXAMPLE 6

### Resin Formulation 6

This resin was prepared in the manner described above from the ingredients set forth in **Table 6** below. The specific ABPU was prepared from the constituent ingredients in the molar ratios indicated in the table in accordance with known techniques such as given in US Patent No. 9,453,142 to Rolland et al.

| **TABLE 6** | |
|---|---|
| **Ingredient** | **Amount** |
| ABPU 14 (IPDI-PTMO 2K-TB, 2.7-1-3.4) | 59.2% |
| NPGDMA | 9.4% |
| UDMA | 9.4% |
| IBOMA | 12.5% |
| TPO | 0.8% |
| DBA | 0.1% |
| MACM | 7.3% |
| D2000 | 1% |
| FD&C Red 40 Lake | 0.0037% |

### EXAMPLE 7

### Production of Sample Objects

The resin formulations described in Examples 1-6 above were placed in the cassette of Carbon Inc. additive manufacturing apparatus and samples were printed. After printing, the samples were cleaned in 95% isopropyl alcohol for 5 minutess on an orbital shaker table, then dried at room temperature. They were then cured in a Dreve PCU-LED UV cure box for 1 minute at a wavelength of 405 nm, under a nitrogen atmosphere. The samples were then baked at 120°C for 4 hours.

### EXAMPLE S

### Tensile Properties of Samples

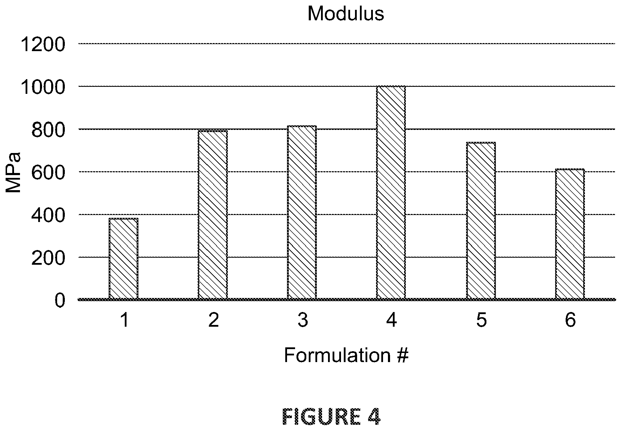

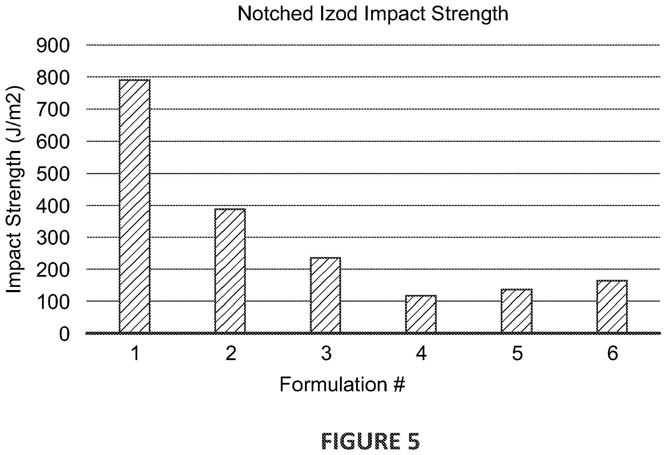

The Modulus of elasticity, and the Notched Izod Impact Resistance, of samples prepared from resin formulations 1-6 were tested in accordance with known techniques. Results are given in **Figures 4-5****.**

**Resin formulation 4** is among those currently preferred because of its high modulus of elasticity (though greater impact strength would in some embodiments be preferred).

**Resin formulation 5** is also among those currently preferred because, although its modulus of elasticity is somewhat lower than that of formulation 4, it was found to be easier to manufacture than resin formulation 4.

The foregoing is illustrative of the present invention, and is not to be construed as limiting thereof. The invention is defined by the following claims

## Claims

1. A method of making a flexible partial denture, comprising:
(*a*) producing a partial denture base (13) by additive manufacturing from a dual cure resin, with the dual cure resin comprising a light polymerizable component and a heat polymerizable component, with the denture base (13) including at least one tooth socket (12) comprising the dual cure resin in uncured or partially cured form;
(*b*) inserting an acrylic artificial tooth (11) into each at least one tooth socket (12) to produce an assembled intermediate product; and then
(*c*) baking said assembled intermediate product for a time sufficient to cure said heat polymerizable component in said denture base (13) and the dual cure resin in uncured or partially cured form in said at least one tooth socket (12), bond said tooth (11) into each at least one socket (12) with said cure, and produce said flexible partial denture.

2. The method of claim 1, wherein the flexible partial denture has a tensile modulus of from 300 to 2000 MPa, and/or an Izod impact strength of from 10 to 500 J/m².

3. The method of claim 1, wherein said method further comprises cleaning the partial denture base (13) prior to the inserting step.

4. The method of claim 1, wherein, prior to or during the inserting step, in the at least one tooth socket (12) the dual cure resin in uncured or partially cured form
- is added to the socket(s) (12) and/or
- is retained dual cure resin in uncured or partially cured form in said at least one socket(s) (12).

5. The method of claim 1, wherein said resin and said denture base (13) are translucent.

6. The method of any preceding claim, said resin comprising a mixture of:
(i) a reactive blocked prepolymer comprising a compound of the formula A-X-A, where X is a hydrocarbyl group and each A is an independently selected substituent of Formula X: 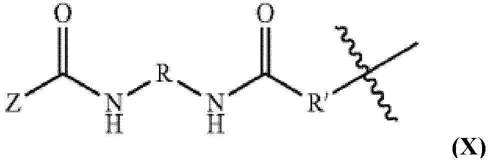 where R is a hydrocarbyl group, R' is O or NH, and Z is a blocking group, said blocking group having a reactive epoxy, alkene, alkyne, or thiol terminal group,
(ii) a chain extender,
(iii) a photoinitiator,
(iv) a reactive diluent,
(v) a reactive blocked diisocyanate,
(vi) optionally, but in some embodiments preferably, a reactive crosslinker;
(vii) optionally, but in some embodiments preferably, at least one pigment or dye; and
(viii) optionally, but in some embodiments preferably, an ultraviolet light absorber.

7. The method of claim 6, wherein said reactive blocked prepolymer comprises a polyisocyanate; and/or said reactive blocked prepolymer comprises two or more ethylenically unsaturated end groups.

8. The method of claim 6 or 7, wherein said reactive blocked prepolymer comprises a polyisocyanate oligomer produced by the reaction of at least one polyisocyanate with at least one polyol or polyamine.

9. The method of any one of claims 6 to 8, wherein said reactive blocked prepolymer is blocked by reaction of a polyisocyanate oligomer with an alcohol (meth)acrylate, amine (meth)acrylate, maleimide, or n-vinylformamide monomer blocking agent.

10. The method of any one of claims 6 to 9, wherein said reactive diluent comprises an acrylate, a methacrylate, a styrene, an acrylic acid, a vinylamide, a vinyl ether, a vinyl ester, polymers containing any one or more of the foregoing, or a combination of two or more of the foregoing.

11. The method of any one of claims 6 to 10, wherein said chain extender comprises at least one diol, diamine or dithiol chain extender.

12. The method of any one of claims 6 to 11, wherein said reactive crosslinker is present and comprises a diacrylate crosslinker.

13. The method of any one of claims 6 to 12, wherein said ultraviolet light absorber is present and comprises polysubstituted linear polyacene that is polysubstituted with substituents independently selected from the group consisting of: bromo, chloro, -Se-R', and -S-R', where each R' is independently selected from alkyl, aryl, and arylalkyl.

14. The method of any one of claims 6 to 12, wherein said ultraviolet light absorber is present and comprises 9,10-dibromoanthracene, 2,3,9,10-tetrabromoanthracene, 5,11-dibromotetracene, or a combination thereof.

15. A flexible partial denture produced by the process of any preceeding claim.

## Patentansprüche

1. Verfahren zur Herstellung einer flexiblen Teilzahnprothese, Folgendes umfassend:
(a) Herstellen einer Teilzahnprothesenbasis (13) durch additive Fertigung aus einem dualhärtenden Harz, wobei das dualhärtende Harz eine lichtpolymerisierbare Komponente und eine wärmepolymerisierbare Komponente umfasst, wobei die Zahnprothesenbasis (13) mindestens ein Zahnfach (12) einschließt, welches das dualhärtende Harz in unausgehärteter oder teilweise ausgehärteter Form umfasst;
(b) Einsetzen eines künstlichen Acrylzahns (11) in jedes mindestens eine Zahnfach (12), um ein zusammengesetztes Zwischenprodukt herzustellen; und anschließend
(c) Brennen des zusammengesetzten Zwischenprodukts über eine Zeit, die ausreicht, um die wärmepolymerisierbare Komponente in der Zahnprothesenbasis (13) und das dualhärtende Harz in unausgehärteter oder teilweise ausgehärteter Form in dem mindestens einen Zahnfach (12) zu härten, den Zahn (11) in jedem mindestens einen Fach (12) mit der Härtung zu verbinden und die flexible Teilzahnprothese herzustellen.

2. Verfahren nach Anspruch 1, wobei die flexible Teilzahnprothese einen Zugmodul von 300 bis 2 000 MPa und/oder eine Izod-Schlagzähigkeit von 10 bis 500 J/m² aufweist.

3. Verfahren nach Anspruch 1, wobei das Verfahren ferner die Reinigung der Teilzahnprothesenbasis (13) vor dem Schritt des Einsetzens umfasst.

4. Verfahren nach Anspruch 1, wobei vor oder während des Schritts des Einsetzens in das mindestens eine Zahnfach (12) das dualhärtende Harz in unausgehärteter oder teilweise ausgehärteter Form
- zu dem Zahnfach oder den Zahnfächern (12) hinzugefügt wird und/oder
- ein in dem mindestens einen Fach (12) zurückgehaltenes dualhärtendes Harz in unausgehärteter oder teilweise ausgehärteter Form ist.

5. Verfahren nach Anspruch 1, wobei das Harz und die Zahnprothesenbasis (13) lichtdurchlässig sind.

6. Verfahren nach einem der vorhergehenden Ansprüche, wobei das Harz eine Mischung aus Folgendem umfasst:
(i) einem reaktiven blockierten Präpolymer, umfassend eine Verbindung der Formel A-X-A, wobei X eine Hydrocarbylgruppe ist und jedes A ein unabhängig ausgewählter Substituent der Formel X ist: 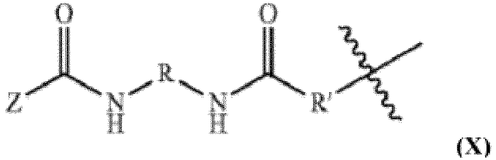 worin R eine Hydrocarbylgruppe ist, R' O oder NH ist und Z eine Blockierungsgruppe ist, wobei die Blockierungsgruppe eine reaktive Epoxy-, Alken-, Alkin- oder Thiol-Endgruppe aufweist,
(ii) einem Kettenverlängerer,
(iii) einem Photoinitiator,
(iv) einem reaktiven Verdünnungsmittel,
(v) einem reaktiven blockierten Diisocyanat,
(vi) wahlweise, in einigen Ausführungsformen jedoch vorzugsweise, einem reaktiven Vernetzer,
(vii) wahlweise, aber in einigen Ausführungsformen vorzugsweise, mindestens einem Pigment oder einem Farbstoff; und
(viii) wahlweise, aber in einigen Ausführungsformen vorzugsweise, einem Ultraviolettlichtabsorber.

7. Verfahren nach Anspruch 6, wobei das reaktive blockierte Präpolymer ein Polyisocyanat umfasst; und/oder das reaktive blockierte Präpolymer zwei oder mehrere ethylenisch ungesättigte Endgruppen umfasst.

8. Verfahren nach Anspruch 6 oder 7, wobei das reaktive blockierte Präpolymer ein Polyisocyanat-Oligomer umfasst, das durch die Reaktion von mindestens einem Polyisocyanat mit mindestens einem Polyol oder Polyamin hergestellt wird.

9. Verfahren nach einem der Ansprüche 6 bis 8, wobei das reaktive blockierte Präpolymer durch Reaktion eines Polyisocyanat-Oligomers mit einem Alkohol(meth)acrylat-, Amin(meth)acrylat-, Maleinimid- oder n-Vinylformamid-Monomerblockierungsmittel blockiert wird.

10. Verfahren nach einem der Ansprüche 6 bis 9, wobei das reaktive Verdünnungsmittel ein Acrylat, ein Methacrylat, ein Styrol, eine Acrylsäure, ein Vinylamid, einen Vinylether, einen Vinylester, Polymere, die eines oder mehrere der Vorgenannten enthalten, oder eine Kombination von zwei oder mehreren der Vorgenannten umfasst.

11. Verfahren nach einem der Ansprüche 6 bis 10, wobei der Kettenverlängerer mindestens einen Diol-, Diamin- oder Dithiol-Kettenverlängerer umfasst.

12. Verfahren nach einem der Ansprüche 6 bis 11, wobei der reaktive Vernetzer vorhanden ist und einen Diacrylat-Vernetzer umfasst.

13. Verfahren nach einem der Ansprüche 6 bis 12, wobei der Ultraviolettlichtabsorber vorhanden ist und polysubstituiertes lineares Polyacen umfasst, das mit Substituenten polysubstituiert ist, die unabhängig voneinander aus der Gruppe ausgewählt sind, bestehend aus Brom, Chlor, -Se-R' und -S-R', wobei jedes R' unabhängig aus Alkyl, Aryl und Arylalkyl ausgewählt ist.

14. Verfahren nach einem der Ansprüche 6 bis 12, wobei der Ultraviolettlichtabsorber vorhanden ist und 9,10-Dibromanthracen, 2,3,9,10-Tetrabromanthracen, 5,11-Dibromtetracen oder eine Kombination davon umfasst.

15. Flexible Teilzahnprothese, hergestellt anhand des Verfahrens nach einem der vorangehenden Ansprüche

## Revendications

1. Procédé de fabrication d'une prothèse dentaire partielle souple, comprenant :
(a) la production d'une base de prothèse dentaire partielle (13) par fabrication additive à partir d'une résine à double durcissement, la résine à double durcissement comprenant un composant polymérisable à la lumière et un composant polymérisable à la chaleur, la base de prothèse dentaire (13) incluant au moins une alvéole de dent (12) comprenant la résine à double durcissement sous forme non durcie ou partiellement durcie,
(b) l'insertion d'une dent artificielle en acrylique (11) dans chaque au moins une alvéole de dent (12) pour produire un produit intermédiaire assemblé, et puis
(c) la cuisson dudit produit intermédiaire assemblé pendant une durée suffisante pour durcir ledit composant polymérisable à la chaleur dans ladite base de prothèse dentaire (13) et la résine à double durcissement sous forme non durcie ou partiellement durcie dans ladite au moins une alvéole de dent (12), lier ladite dent (11) dans chaque au moins une alvéole (12) par ledit durcissement, et produire ladite prothèse dentaire partielle souple.

2. Procédé selon la revendication 1, dans lequel la prothèse dentaire partielle souple présente un module de traction de 300 à 2 000 MPa, et/ou une résistance au choc Izod de 10 à 500 J/m².

3. Procédé selon la revendication 1, dans lequel ledit procédé comprend en outre le nettoyage de la base de prothèse dentaire partielle (13) avant l'étape d'insertion.

4. Procédé selon la revendication 1, dans lequel, avant ou pendant l'étape d'insertion, dans l'au moins une alvéole de dent (12), la résine à double durcissement sous forme non durcie ou partiellement durcie
- est ajoutée à l'alvéole ou aux alvéoles (12) et/ou
- est une résine à double durcissement retenue sous forme non durcie ou partiellement durcie dans ladite au moins une alvéole (12).

5. Procédé selon la revendication 1, dans lequel ladite résine et ladite base de prothèse dentaire (13) sont translucides.

6. Procédé selon l'une quelconque des revendications précédentes, ladite résine comprenant un mélange des suivants :
(i) un pré-polymère bloqué réactif comprenant un composé de formule A-X-A, où X est un groupe hydrocarbyle, et chaque A est un substituant indépendamment sélectionné de formule X : 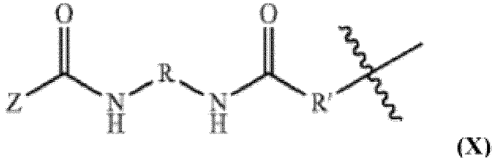 où R est un groupe hydrocarbyle, R' est O ou NH, et Z est un groupe bloquant, ledit groupe bloquant comportant un groupe terminal réactif époxy, alcène, alcyne ou thiol,
(ii) un allongeur de chaîne,
(iii) un photo-amorceur,
(iv) un diluant réactif,
(v) un diisocyanate bloqué réactif,
(vi) facultativement, mais de préférence dans certains modes de réalisation, un agent de réticulation réactif,
(vii) facultativement, mais de préférence dans certains modes de réalisation, au moins un pigment ou un colorant, et
(viii) facultativement, mais de préférence dans certains modes de réalisation, un absorbant de lumière ultraviolette.

7. Procédé selon la revendication 6, dans lequel ledit pré-polymère bloqué réactif comprend un polyisocyanate, et/ou ledit pré-polymère bloqué réactif comprend deux ou plusieurs groupes terminaux éthyléniquement insaturés.

8. Procédé selon la revendication 6 ou 7, dans lequel ledit pré-polymère bloqué réactif comprend un oligomère de polyisocyanate produit par la réaction d'au moins un polyisocyanate avec au moins un polyol ou un polyamine.

9. Procédé selon l'une quelconque des revendications 6 à 8, dans lequel ledit pré-polymère bloqué réactif est bloqué par la réaction d'un oligomère de polyisocyanate avec un agent monomère bloquant de (méth)acrylate d'alcool, (méth)acrylate d'amine, maléimide, ou de n-vinylformamide.

10. Procédé selon l'une quelconque des revendications 6 à 9, dans lequel ledit diluant réactif comprend un acrylate, un méthacrylate, un styrène, un acide acrylique, un vinylamide, un éther de vinyle, un ester de vinyle, des polymères contenant un ou plusieurs des précédents, ou une combinaison de deux ou plusieurs des précédents.

11. Procédé selon l'une quelconque des revendications 6 à 10, dans lequel ledit allongeur de chaîne comprend au moins un allongeur de chaîne diol, diamine ou dithiol.

12. Procédé selon l'une quelconque des revendications 6 à 11, dans lequel ledit agent de réticulation réactif est présent et comprend un agent de réticulation diacrylate.

13. Procédé selon l'une quelconque des revendications 6 à 12, dans lequel ledit absorbant de lumière ultraviolette est présent et comprend un polyacène linéaire polysubstitué qui est polysubstitué par des substituants sélectionnés indépendamment parmi le groupe constitué de bromo, chloro, -Se-R' et -S-R', où chaque R' est sélectionné indépendamment parmi un alkyle, aryle, et arylalkyle.

14. Procédé selon l'une quelconque des revendications 6 à 12, dans lequel ledit absorbant de lumière ultraviolette est présent et comprend du 9,10-dibromoanthracène, du 2,3,9,10-tétrabromoanthracène, du 5,11-dibromotétracène ou une combinaison de ceux-ci.

15. Prothèse dentaire partielle souple produite par le procédé selon l'une quelconque des revendications précédentes.
